# EUROPEAN PATENT APPLICATION

(11) **EP 3 851 040 A1**
(43) Date of publication of application: **21.07.2021**
(21) Application number: 20305032.3
(22) Date of filing: 16.01.2020
(51) Int. Cl.: A61B 5/145, G01N 27/327, A61M 37/00

(54) **BODY MONITORING DEVICE AND ASSOCIATED METHOD**

(71) Applicant: PKvitality, 75001 Paris (FR)
(72) Inventor: PIERART, Luc, 94800 VILLEJUIF (FR); PEACOCK, Martin, Royston, Hertfordshire SG8 5HB (GB)
(74) Representative: Regimbeau

(57) **Abstract**

The present invention relates to a method of a method of measuring a human body analyte concentration in an interstitial fluid, comprising the step of measuring a first electrical current I₁ between a working electrode and a pseudo-reference electrode while applying a first potential between the working electrode and the pseudo-reference electrode, the first potential being less than a threshold potential, the magnitude of the first electrical current being greater than the magnitude of a predetermined first threshold current, and further measuring an output electrical current between the working electrode and the pseudo-reference electrode while applying a second electrical potential, the second electrical potential being greater than the first electrical potential.

## Description

### TECHNICAL FIELD

The present invention relates to a device for measuring a body analyte. More precisely, it concerns a body monitoring device configured for analyzing a body fluid, typically an interstitial body fluid as glucose.

### BACKGROUND OF THE INVENTION

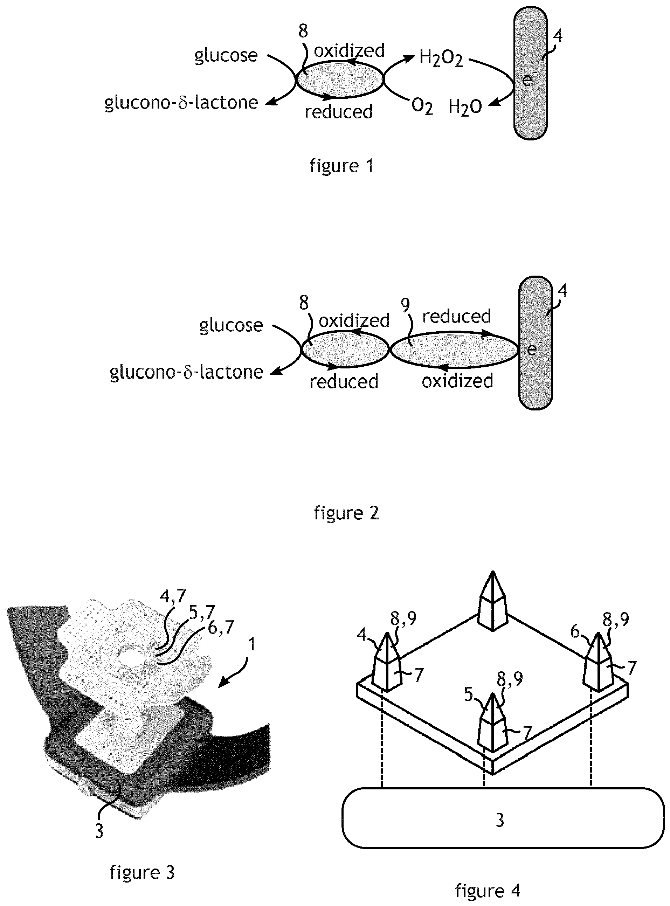

With reference to figure 1, it is known to measure the concentration of glucose, by using the reactivity of the glucose oxidase (GOx) with its natural electron collector O₂. Clark *et al. (*Clark Jr, L. C., & Lyons, C., 1962, Electrode systems for continuous monitoring in cardiovascular surgery, Annals of the New York Academy of sciences, 102(1), 29-45*)* disclose an amperometric device, comprising an anode and a cathode, configured for monitoring a glucose concentration. When glucose is present in the fluid to be measured, GOx oxides the glucose while reducing O₂, according to the following the equation:

glucose + 0₂ → gluconolactone + H₂O₂ (1)

The O₂ depletion induces a decrease in the reduction current of the O₂ on the cathode, according to the following equation:

0₂ + 4 e⁻ + 2 H₂O → 4 OH⁻ (2)

The decrease in the reduction current is proportional to the concentration of glucose in the body fluid.

However, the current that the cathode would deliver in the absence of glucose has to be determined by other means, for example by measuring the current of a control device.

Therefore, it is known to overcome this limitation by measuring an oxidation current of the hydrogen peroxide produced in the reaction of equation 1. The oxidation of H₂O₂ can be performed on a platinum electrode by controlling the potential difference between the cathode and the anode at a potential of 0,6 V, according to the following equation:

H₂O₂ → O2 + 2 H+ + 2 e- (3)

However, when a potential over 400 mV, and more particularly over 650 mV is applied between the electrodes, several species, called interferent species, naturally present in a body fluid, such as ascorbate (vitamin C) or urate, can oxidize on the platinum electrode, and induce noise in the electric measurement. Also, some exogenous substances can oxidize on the platinum electrode. In addition to interacting on the measurement through the corresponding electron transfer, this oxidation tends to damage the electrode and therefore on the device lifetime.

With reference to figure 2, in order to overcome the above-mentioned disadvantages, it is known to get rid of the contribution of O₂ when measuring glucose by using artificial redox mediators.

However, the use of a redox mediator leads to a saturation of the redox current between the electrodes when measuring high analyte concentrations, for example concentrations of analyte above 11 mM. The saturation degrades the precision of the concentration measurement at high analyte concentrations.

### SUMMARY OF THE INVENTION

A method of measuring a human body analyte in an interstitial fluid of a user by a device has been developed to respond at least partially to the above-mentioned issues of the prior art.

An aspect of the invention is a method of measuring a human body analyte concentration in an interstitial fluid of a user by a device comprising:
- a control unit,
- at least a working electrode and a pseudo-reference electrode, the working electrode and the pseudo-reference electrode being configured for an implantation in the dermis of the user by at least one microneedle so as to be in contact with the interstitial fluid of the user,
- an enzyme configured for oxidizing the analyte, the enzyme being attached over at least a part of the working electrode,
- a mediator being fixed on said part of the working electrode, the method comprising the step of:
   a) measuring a first electrical current I₁ between the working electrode and the pseudo-reference electrode while applying a first potential V₁ between the working electrode and the pseudo-reference electrode, the first potential V₁ being less than a predetermined threshold potential Vₜ, the magnitude of the first electrical current I₁ being greater than the magnitude of a predetermined first threshold current Iₜ₁, and further measuring an output electrical current I₀ between the working electrode and the pseudo-reference electrode while applying a second electrical potential V₂, the second electrical potential V₂ being greater than the first electrical potential V₁, and/or comprising the step of
   b) measuring a second electrical current I₂ between the working electrode and the pseudo-reference electrode while applying a third electrical potential V₃ between the working electrode and the pseudo-reference electrode, the third potential V₃ being greater than the threshold potential Vₜ, the magnitude of the second electrical current I₂ being less than a magnitude of a predetermined second threshold current Iₜ₂, and then measuring the output electrical current I₀ while applying a fourth electrical potential V₄, the fourth electrical potential V₄ being inferior to the third electrical potential V₃.

In further optional aspects of the invention:
- the mediator is a redox mediator having a reduced state and an oxidized state, and being configured for being in a reduced state below the predetermined threshold electrical potential Vₜ, and in an oxidized state above the predetermined threshold electrical potential Vₜ,
- the second electrical potential V₂ is greater than the threshold electrical potential Vₜ and/or the fourth electrical potential V₄ is under the threshold electrical potential Vₜ,
- the method comprises both step a) and step b),
- step a) and/or step b) comprise(s) a sub-step of computing a concentration of the analyte in the interstitial fluid from the value of the output electrical current Iₒ,
- the first threshold current Iₜ₁ and the second threshold Iₜ₂ current are of opposite signs,
- comprises a repetition of the steps of measuring the current between the working electrode and the pseudo-reference electrode and determining a concentration of the analyte in the interstitial fluid from the value of the output electrical current I₀,
- each repetition is separated by a time between 0.5 s and 30 min, and preferably between 15 s and 15 min,
- the threshold potential Vₜ is comprised between 10 mV and 650 mV,
- the first threshold current Iₜ₁ is comprised in the range from - 500 nA and
- 0,1 nA and preferably between - 200 nA and -0,1 nA,
- the human body analyte is at least chosen between glucose and lactate,
- the mediator is Prussian blue.

Another aspect of the invention is device for measuring a human body analyte in an interstitial fluid of a user by a device comprising:
- a control unit,
- at least a working electrode and a pseudo-reference electrode, the working electrode and the pseudo-reference electrode being configured for an implantation in the dermis of the user by at least one microneedles so as to be in contact with the interstitial fluid of the user,
- an enzyme configured for oxidizing the analyte, the enzyme being attached over at least a part of the working electrode,
- a mediator being fixed on said part of the working electrode,
   the control unit being configured for:
   a) measuring a first electrical current I₁ between the working electrode and the pseudo-reference electrode while applying a first potential V₁ between the working electrode and the pseudo-reference electrode, the first potential V₁ being less than a predetermined threshold potential Vₜ, the magnitude of the first electrical current I₁ being greater than the magnitude of a predetermined first threshold current Iₜ₁, and further measuring an output electrical current I₀ between the working electrode and the pseudo-reference electrode while applying a second electrical potential V₂, the second electrical potential V₂ being greater than the first electrical potential V₁, and/or for
   b) measuring a second electrical current I₂ between the working electrode and the pseudo-reference electrode while applying a third electrical potential V₃ between the working electrode and the pseudo-reference electrode, the third potential V₃ being greater than the threshold potential Vₜ, the magnitude of the second electrical current I₂ being less than a magnitude of a predetermined second threshold current Iₜ₂, and then measuring the output electrical current I₀ while applying a fourth electrical potential V₄, the fourth electrical potential V₄ being inferior to the third electrical potential V₃.

### DEFINITIONS

The term "microneedle" is used herein to designate a needle having a maximum length between 10 µm and 1 mm.

The terms "pseudo reference electrode" will be used herein to designate a reference electrode or a counter electrode used as a reference electrode in the absence of reference electrode.

The terms "mediator" will be used herein to designate a species adapted to mediate electrons from an electrode to a chemical species by oxidoreduction (mediator) or to catalyze the electron transfer between the electrode and said chemical species. Both kind of mediators (redox mediator and catalyst) can be used in the invention.

### BRIEF DESCRIPTION OF THE DRAWINGS

The invention will be described by way of example, with reference to the accompanying drawings in which:
- figure 1 diagrammatically shows a reaction scheme of a first-generation glucose biosensor of the prior art, in which electrons are transferred from the enzyme to the electrode by the oxidation of H₂O₂,
- figure 2 diagrammatically shows a reaction scheme of a second-second generation glucose biosensor of the prior art, in which electrons are transferred from the enzyme to the electrode by the oxidoreduction of a mediator,
- figure 3 diagrammatically shows a device according to an embodiment of the invention,
- figure 4 diagrammatically shows a device according to an embodiment of the invention,
- figure 5A diagrammatically shows a reaction scheme adapted to detect an analyte according to an embodiment of the invention,
- figure 5B diagrammatically shows a reaction scheme adapted to detect an analyte according to an embodiment of the invention,
- figure 6 diagrammatically shows a method according to an embodiment of the invention.

### DETAILED DESCRIPTION OF PREFERRED ASPECTS OF THE INVENTION

### General architecture of the device 1

In reference with figure 3 and figure 4, a device 1 for measuring a human body analyte 2 in an interstitial fluid of a user comprises a working electrode 4, a counter electrode 5 (also called auxiliary electrode) and preferably a reference electrode 6.

The electrodes (*i.e.* the working electrode 3, the counter electrode 4 and preferably the reference electrode 6) are configured to be implanted in the dermis of a user by one or several microneedles 7 so as to be in contact with the interstitial fluid of the user. For example, the microneedle 7 can be metallic, so that the microneedle is the electrode. Metallic track can also be deposited on a microfabricated microneedle 7 so as to form the electrode. In general, the microneedle 7 has a metallic surface S which can be electrically connected outside of the microneedle 7. Several electrodes can be fabricated on the same microneedle 7, by electrically insulating the metallic surface of each electrode from the other metallic surface(s). In another embodiment of the invention, each electrode can be mounted on a different microneedle 7. With reference to figure 4, each electrode can have a basis and a top. The metallic surface of each electrode can at least have a part arranged at a distance superior to 100 µm and preferably 300 µm from the basis of the electrode. Therefore, a part of the electrode is not in contact with the epidermis, which does not contain available interstitial fluid, but with the dermis, which contains interstitial fluid. Preferably, the top of each microneedle 7 is arranged at a distance inferior to 1 mm from the basis of the electrode. Therefore, the microneedle is short enough to avoid contacting a nerve and causing pain to the user.

The device 1 also comprises a control unit 3. The control unit 3 is electrically connected to the electrodes, particularly to the working electrode 4, to the counter electrode 5, and preferably to the reference electrode 6. The control unit 3 can comprise processor, a memory, an electrical acquisition module and an electrical control module. Preferably, every working electrode 4 and counter electrode 5 is independently electrically connected to the electrical acquisition module and to the electrical control module. The electrical connections are illustrated in figure 4 by dashed lines.

### Electrode surface treatment

The device 1 comprises enzymes 8 configured for oxidizing the analyte 2. Each enzyme 8 comprises a cofactor which is responsible for the oxidation of the analyte, preferably glucose or lactate. The enzyme 8 is preferably an enzyme chosen between glucose oxidase and dehydrogenase, such as PQQ-glucose dehydrogenase, NAD-glucose dehydrogenase or FAD-glucose dehydrogenase. The enzymes 8 are attached over at least a part of the working electrode. The enzymes 8 can be attached over the part of the electrode for example by adsorption, by covalent grafting, by cross-linking and/or by encapsulation in a matrix.

The device 1 comprises mediators. The mediator is attached at least over the part of the working electrode on which the enzymes are attached. The mediator is preferably chosen between Prussian blue, a conducting polymer, an osmium complex, and a cobalt complex. In a preferred embodiment of the invention, the mediator is Prussian blue (having the chemical formula Fe^{III}₄[Fe^{II}(CN)₆]₃).

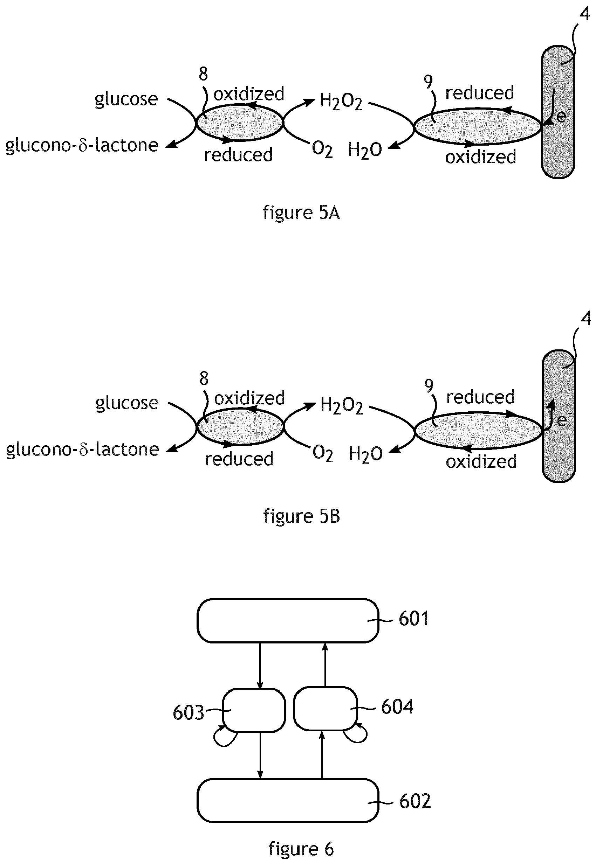

With reference to figure 5A and figure 5B, the mediator, preferably Prussian blue, allows both oxidation and reduction of H₂0₂ for respectively transferring electrons or capturing electrons from the electrode it is fixed on.

With reference to figure 5A, for an applied voltage being under an applied threshold potential Vₜ between the working electrode 4 and a pseudo-reference electrode, said threshold potential being chosen between 20 mV and 650 mV, the mediator 9 is configured for reducing H₂O₂ which is produced by the enzyme 8. Then, the mediator 9, which is in an oxidized state, can be reduced at the working electrode 4. The working electrode gives electrons to the mediator 9, and a negative current takes place between the working electrode 4and the pseudo-reference electrode.

With reference to figure 5B, for an applied voltage being over an applied threshold potential Vₜ between the working electrode and a pseudo-reference electrode, said threshold potential being chosen between 20 mV and 650 mV, the mediator 9 is configured for oxidizing H₂O₂ which is produced by the enzyme 8. Then, the mediator 9, which is in a reduced state, can be oxidized at the working electrode 4. The working electrode 4 takes electrons from the mediator 9, and a positive current takes place between the working electrode 4 and the pseudo-reference electrode.

A porous membrane can, for example, be deposited on the metallic surface of the electrode. Glucose oxidase enzymes 8 can be, for example, grafter on the membrane. Then, Prussian blue can be deposited on both the membrane and the glucose oxidase enzymes 8.

The surface *S* of the working electrode 4 covered with both the enzymes 8 and the mediators 9 is preferably comprised between 20 000 µm² and 600 000 µm².

### Analyte 2 measurement

With reference to figure 5 and figure 6, an aspect of the invention is a method of measuring a human body analyte 2 in an interstitial fluid of a user by a device, and preferably measuring the concentration of said body analyte 2.

After inserting the microneedles 7 of the device 1 in the skin of the user, more specifically in the dermis of the user, the method comprises:
- a step 601 of measuring a first electrical current I₁ between the working electrode 4 and the pseudo-reference electrode 5 while applying a first potential V₁ between the working electrode 4 and the pseudo-reference electrode 5, the first potential V₁ being less than the threshold potential Vₜ, the magnitude of the first electrical current I₁ being greater than the magnitude of a predetermined first threshold current Iₜ₁, and further measuring an output electrical current I₀ between the working electrode 4 and the pseudo-reference electrode 5 while applying a second electrical potential V₂, the second electrical potential V₂ being greater than the first electrical potential V₁, or
- a step 602 of b) measuring a second electrical current I₂ between the working electrode 4 and the pseudo-reference electrode 5 while applying a third electrical potential V₃ between the working electrode 4 and the pseudo-reference electrode 5, the third potential V₃ being greater than the threshold potential Vₜ, the magnitude of the second electrical current I₂ being less than a magnitude of a predetermined second threshold current Iₜ₂, and then measuring the output electrical current I₀ while applying a fourth electrical potential V₄, the fourth electrical potential V₄ being inferior to the third electrical potential V₃.

Therefore, it is possible to switch the predominant reaction path in order to transfer electrons from the enzymes 8 to the working electrode 4, depending on the concentration of analyte 2 in the interstitial fluid.

Specifically, step 601 allows to switch from a first potential V₁ adapted to measure low concentrations of analytes 2 to a second potential V₂ adapted to measure higher concentrations of analyte 2. The terms "low concentration" are used herein to define a concentration under 11 mM, and preferably than 7 mM. Typically, in order to be accurate when measuring low concentrations of analyte 2, the electron transfer implying a reduction of H₂O₂ and a reduction of the mediator at the electrode is adapted because it allows to avoid a contribution of interferent species in the measurement. When the concentration increases, it leads the magnitude of the current to increase. Over a magnitude of a predetermined first threshold current Iₜ₁, this chemical pathway suffers from non-linearity and saturation. Then, a second potential V₂, greater than V₁, is applied between the working electrode and the pseudo-reference electrode in order to foster the chemical pathway adapted to high concentrations of analyte 2.

Step 602 allows to switch from a third potential V₃ adapted to measure high concentrations of analytes 2 to a fourth electrical potential V₄, adapted to measure lower concentrations of analyte 2. The terms "high concentration" are used herein to define a concentration of analyte 2 greater than 11 mM, and preferably than 13 mM. Typically, in order to be accurate when measuring high concentrations of analyte 2, the electron transfer implying a oxidation of H₂O₂ and an oxidation of the mediator 9 at the electrode, or a direct oxidation of H₂O₂ at the working electrode 4, is adapted because it avoids limiting the quantity of current due to the mediator 9 quantity at the electrode. When the concentration of analyte 2 decreases, it leads the magnitude of the current to decrease. Under a magnitude of a predetermined second threshold current Iₜ₂, this chemical pathway suffers from noise of the interferent species also reacting at this potential. Then, a fourth potential V₄, lower than V₃, is applied between the working electrode and the pseudo-reference electrode in order to foster the chemical pathway adapted to low concentrations of analyte 2.

The method can preferably comprise both steps 601 and 602, allowing to perform a continuous measurement of analyte 2 with the most favorable chemical pathway.

The method preferably comprises a repetition of steps 603 and/or steps 604, each step comprising measuring the current I₀ between the working electrode 4 and the pseudo-reference electrode, and determining a concentration of the analyte 2 in the interstitial fluid from the value of the said current. The determination of the concentration can be done using predetermined calibration data, or simultaneous measurement of a reference analyte 2 concertation. Step 603 comprises measuring the output current I₀ while applying an electrical potential V₂, the electrical potential V₂ being greater than the potential V₁. Step 604 comprises measuring the output current I₀ while applying an electrical potential V₃, the electrical potential V₃ being lower than the potential V₄.

Preferably, step 603 or step 604 is repeated at a time between 10 s and 30 min, and preferably between 15 s and 15 min. Therefore, it is possible to record pseudo-continuously the analyte 2 concentration while both having the most precise reaction pathway to measure the analyte concentration.

The surface S of the working electrode 4 is preferably comprised between 20 000 µm² and 600 000 µm². Therefore, it is possible to limit the electrical noise when measuring the output current Iₒ while minimizing the size of the microneedle 7 to reduce the pain of the user. Conversely, as the surface S is limited, the conditions of the step 601 and/or 602 can be expressed in terms of current density.

Because of different electron pathways, depending on the applied electrical potential between the working electrode 4 and the pseudo-reference electrode, the presence of analyte can induce an oxidation between the metal of the working electrode and another species. Hence, the current measured between the electrodes at step 603 and step 604 can be of opposite signs. Moreover, the first threshold current and the second threshold current can of opposite signs.

Experimentally, the inventors measured that the first threshold current Iₜ₁ is preferably comprised between - 500 nA and -1 nA and, notably between - 200 nA and -1 nA. The second current threshold is preferably comprised between 50 nA and 1 µA, notably between 100 nA and 1 µA.

The threshold potential Vₜ is preferably comprised between 10 mV and 650 mV, notably between 100 mV and 400 mV.

## Claims

1. A method of measuring a human body analyte (2) concentration in an interstitial fluid of a user by a device (1) comprising:
- a control unit (3),
- at least a working electrode (4) and a pseudo-reference electrode (5), the working electrode (4) and the pseudo-reference electrode (5) being configured for an implantation in the dermis of the user by at least one microneedle (7) so as to be in contact with the interstitial fluid of the user,
- an enzyme (8) configured for oxidizing the analyte (2), the enzyme (8) being attached over at least a part of the working electrode,
- a mediator (9) being fixed on said part of the working electrode (4), the method comprising the step of:
a) measuring a first electrical current I₁ between the working electrode (4) and the pseudo-reference electrode (5) while applying a first potential V₁ between the working electrode (4) and the pseudo-reference electrode (5), the first potential V₁ being less than a predetermined threshold potential Vₜ, the magnitude of the first electrical current I₁ being greater than the magnitude of a predetermined first threshold current Iₜ₁, and further measuring an output electrical current I₀ between the working electrode (4) and the pseudo-reference electrode (5) while applying a second electrical potential V₂, the second electrical potential V₂ being greater than the first electrical potential V₁,
and/or comprising the step of
b) measuring a second electrical current I₂ between the working electrode (4) and the pseudo-reference electrode (5) while applying a third electrical potential V₃ between the working electrode (4) and the pseudo-reference electrode (5), the third potential V₃ being greater than the threshold potential Vₜ, the magnitude of the second electrical current I₂ being less than a magnitude of a predetermined second threshold current Iₜ₂, and then measuring the output electrical current I₀ while applying a fourth electrical potential V₄, the fourth electrical potential V₄ being inferior to the third electrical potential V₃.

2. The method of claim 1, wherein the mediator (9) is a redox mediator (9) having a reduced state and an oxidized state, and being configured for being in a reduced state below the predetermined threshold electrical potential Vₜ, and in an oxidized state above the predetermined threshold electrical potential Vₜ.

3. The method of claim 1 or 2, wherein the second electrical potential V₂ is greater than the threshold electrical potential Vₜ and/or the fourth electrical potential V₄ is under the threshold electrical potential Vₜ.

4. The method according to any of claims 1 to 3, comprising both step a) and step b).

5. The method according to any of claims 1 to 4, wherein step a) and/or step b) comprise(s) a sub-step of computing a concentration of the analyte in the interstitial fluid from the value of the output electrical current Iₒ.

6. The method according to any of claims 1 to 5, wherein the first threshold current Iₜ₁ and the second threshold Iₜ₂ current are of opposite signs.

7. The method according to any of claims 1 to 6, comprising a repetition of the steps of measuring the current between the working electrode (4) and the pseudo-reference electrode and determining a concentration of the analyte (2) in the interstitial fluid from the value of the output electrical current I₀.

8. The method according to claim 7, wherein each repetition is separated by a time between 0.5 s and 30 min, and preferably between 15 s and 15 min.

9. The method according to claim 8, wherein the threshold potential Vₜ is comprised between 10 mV and 650 mV.

10. The method according to claims 1 to 9, wherein the first threshold current Iₜ₁ is comprised in the range from - 500 nA and -0,1 nA and preferably between - 200 nA and -0,1 nA.

11. The method according to any of claims 1 to 10, wherein the human body analyte is at least chosen between glucose and lactate.

12. The method according to any of claims 1 to 11, wherein the mediator (9) is Prussian blue.

13. A device for measuring a human body analyte (2) in an interstitial fluid of a user by a device (1) comprising:
- a control unit (3),
- at least a working electrode (4) and a pseudo-reference electrode (5), the working electrode (4) and the pseudo-reference electrode (5) being configured for an implantation in the dermis of the user by at least one microneedles (7) so as to be in contact with the interstitial fluid of the user,
- an enzyme (8) configured for oxidizing the analyte (2), the enzyme (8) being attached over at least a part of the working electrode,
- a mediator (9) being fixed on said part of the working electrode (4),
the control unit being configured for:
a) measuring a first electrical current I₁ between the working electrode (4) and the pseudo-reference electrode (5) while applying a first potential V₁ between the working electrode (4) and the pseudo-reference electrode (5), the first potential V₁ being less than a predetermined threshold potential Vₜ, the magnitude of the first electrical current I₁ being greater than the magnitude of a predetermined first threshold current Iₜ₁, and further measuring an output electrical current I₀ between the working electrode (4) and the pseudo-reference electrode (5) while applying a second electrical potential V₂, the second electrical potential V₂ being greater than the first electrical potential V₁,
and/or for
b) measuring a second electrical current I₂ between the working electrode (4) and the pseudo-reference electrode (5) while applying a third electrical potential V₃ between the working electrode (4) and the pseudo-reference electrode (5), the third potential V₃ being greater than the threshold potential Vₜ, the magnitude of the second electrical current I₂ being less than a magnitude of a predetermined second threshold current Iₜ₂, and then measuring the output electrical current I₀ while applying a fourth electrical potential V₄, the fourth electrical potential V₄ being inferior to the third electrical potential V₃.
